# EUROPEAN PATENT SPECIFICATION

(11) **EP 0 376 920 B1**
(45) Date of publication and mention of the grant of the patent: **31.05.1995**
(21) Application number: 90100049.7
(22) Date of filing: 02.01.1990
(51) Int. Cl.: G01N 33/28

(54) **Method and arrangement for diagnostics of friction systems of motors**
Verfahren und Vorrichtung zur Untersuchung von Reibungssystemen in Motoren
Méthode et appareil pour l'examen de systèmes à friction dans les moteurs

(30) Priority: 30.12.1988 CS 9111/88; 30.12.1988 CS 9113/88
(43) Date of publication of application: 04.07.1990
(73) Proprietor: Novotny, Ladislav, 100 00 Praha 10 (CZ)
(72) Inventor: Novotny, Ladislav, Dipl.Ing. CSc, Praha 10 (CS); Kalvoda, Robert, Doz. DrSc, Praha 6 (CS); Novak, Vladimir, Doksy (CS); Uher, Karel, Doksy (CS)
(74) Representative: Beetz & Partner Patentanwälte

(56) References cited:
- DE-B- 1 175 463
- FR-A- 2 513 412
- GB-A- 2 175 092
- US-A- 3 526 127
- PATENT ABSTRACTS OF JAPAN, vol. 6, no. 188 (P-144)[1066], 28 September 1982

## Description

This invention relates to a method for diagnostic of friction systems of motors based on analysis of unused and used lubricant according to the first part of claim 1 and 3, resp., and to an apparatus for carrying out this method.

For the judging the technical state of a motor or another mechanical system there are known some methods, in which a sample of the lubrication oil of said motor, especially of an internal combustion engine, will be proved of the content of abrasive metal particles. In such a method, described in the GB-A-2 175 092, an inductance coil situated around a permanent magnet are used as probe for insertion into an oil gear box for the metering of metallic debris therein. Since the ferrous particles are captured by the permanent magnet, the electric inductance of the coils changes due to the proximity of the particles. The amount of said particles is determined by the changes of the operating frequency of a band-pass filter.

The US-3 526 127 discloses a system for analyzing the various characteristics of a plurality of lubrication oil samples used in motors and engines. A computer analyzes and inter-relates data about the viscosity, the infra-red characteristics and the metal content of said oil samples and provides output data corresponding to the condition of the engine and of the lubrication oil.

Further known methods for analyzing the lubrication oil of a motor, an internal combustion engine or other mechanical systems use the atomic emission spectrography (AES) and the atomic absorption flame spectrometry (AAS). In the mostly preferred AAS-method a sample of oil will be filtered and diluted by an appropriate solvent and thereafter is burnt in a special burner while the absorption spectrum is measured by a highly sensitive apparatus. In spite of high cost, operations complications, unfitness for current workshop conditions and other limitations the spectral tribodiagnostics is the widely applied method of objective triboanalysis, used especially in USA, Canada and Western Europe. There exist other methods of technical diagnostics like vibration and acoustic method and methods based on estimation of combustion products. Their main disadvantage is that they indicate only the critical states of the motors being not sufficiently sensitive and exact. For the lack of relatively cheap, sensitive and simple methods and arrangements for objective tribodiagnostics without dismantling motors the prevailing methods in majority of countries are costly and laborious methods requiring dismantling.

One of the limitations and drawbacks of the above spectral tribodiagnostic methods is the circumstance that the informations concern the total concentration of the elements and not the form in which they occur, like their oxidation states. They do not allow a determination of organic components of the samples or a detection of changes in lubricants. Further for some non-primary metals, as lead, their sensitivity is limited, and their direct applicability for analysis of dispersed abraded particles larger than 10 »m is not possible. Disadvantageous are also the relatively high cost, the restricted use in current workshop conditions, limited precision, bulkiness, etc.

An object of the invention is to provide a method for diagnostics of friction systems of motors, which is simple in use, highly sensitive under different workshop conditions and which gives the possibility for determining the extension of wear of the motor or the friction system.

Said object will be solved by the features of the claims 1 and 3, resp.

A further object of the invention is to provide an apparatus for carrying out such a method for diagnostics of the friction systems of motors, which is easy to handle, has a light weight and supplies exact information about the conditions of the friction system by low costs.

Said object will be solved according to the invention by the features of claim 5.

The method of diagnostics of motors according to the invention widens considerably the scope of objective tests of the state of combustion engines and other motors in view of detection and timely repair of minor defects, timely planned major repairs and avoiding unexpected break-downs. Providing that the diagnostic system is manufactured in a high standard, new possibilities will open for the repair and maintenance practice, as the financial accessibility of the present method is more than 10 times more favourable than the spectral methods.

### EXAMPLE N° 1

An example of application of the present invention is an estimation of the degree of wear of an internal combustion engine which is done as follows. From the lubrication system of the motor warmed-up to the usual operating temperature a sample of lubrication oil is taken. 5 g of this oil after addition of 3 ml of solvent based on chlorinated hydrocarbons is covered by 5 ml of hydrochloric acid diluted in proportion 2:1 and an extraction of the microparticles from oil into the aqueous solution is carried out for 15 minutes. Then after addition of 45 ml of water the extraction is continued for 10 minutes more. After a separation of phases, 10 ml of the aqueous phase are mixed with 5 ml of 6M potassium hydroxide and after a dissolution of 2-3 g of sodium sulphite in said mixture the resulting solution is placed in a cell. After connecting a source of linearly increasing voltage to a measuring mercury electrode and a reference silver chloride electrode a current-voltage curve is recorded in a potential range of -0.24 to -1.70 V at a scan rate of 20 mV/sec. The measuring electrode is realized as a droplet of mercury having a radius of about 0.3 mm formed in a given moment at an orifice of a special capillary by delivery of an appropriate micro-amount of mercury from a mercury reservoir. After recording the above curve 10 ml of 0.1N triethanolamine are added to the solution in the cell and after stirring a current-voltage curve is recorded in a range of -0.6 V to -1.2 V. From the course and by a comparison of both recordings the concentration of copper, lead, zinc and iron in the motor oil is determined and by means of calibration data the extent of wear of the friction system of the oil combustion engine is estimated.

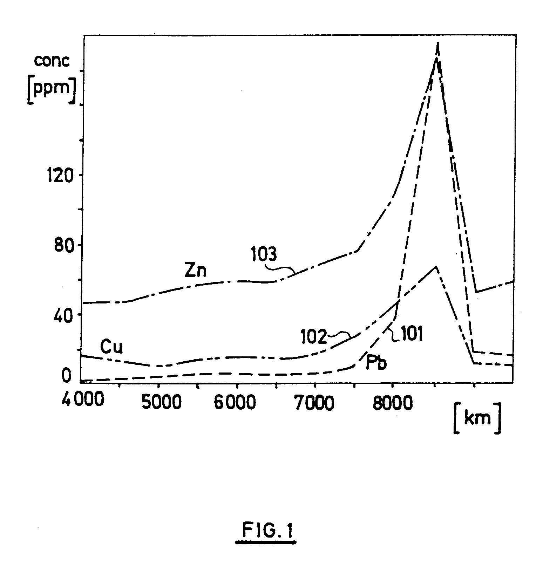

The magnitudes of the measured signals or of the determined concentrations are plotted into graphs, e.g., as a function of mileage covered by the vehicle with the motor which is being followed. An increase of the measured signals or of the determined concentrations over a certain level indicates the accurrence, the cause or even the possible location of a failure. Fig. 1 shows a graph of the dependence of the concentration of copper (curve 102), lead (curve 101) and zinc (curve 103) in the extraction solution as a function of the milage of an automobile equipped with the combustion engine. The exponential increase of concentration of these metals indicates an unacceptable wear of bearings. The position of maxima on the curves corresponds to the state of the motor before its break-down, the steep decrease to the state after repair.

### EXAMPLE N° 2

A sample of 5 g of used lubrication oil is taken from the lubricating system of the motor. After an addition of 5 ml of a solvent based on chlorinated hydrocarbons to said oil sample the solution is extracted for 5 minutes by 5 ml of 3M HCl and then a sample of 5 ml of aqueous extract is taken. Into this aqueous sample sodium pyrophosphate and triethanolamine are added up to a concentration of 0.1 mol/l. Then the acidity of the sample is adjusted by addition of 1M NaOH to pH = 9, whereupon the measuring electrode based on mercury drop and the silver chloride reference electrode are dipped into the sample of the extract. A voltage is applied to the measuring electrode and to the reference electrode linearly increasing from -0.1 to -1.5 V at the rate of 50 mV/s while a current-voltage curve is recorded. From the hights of peaks or waves formed in the region of -0.3 V and -0.9 V the concentration of ions of iron in the valency II and III is determined and from the data thus gained and compared with standard samples the abrasive properties of the metal present in the used oil can be determined as well as the total wear of the motor, the cylinder struts, the pistons, etc. The measured signals are plotted in graphs in dependence on covered milage.

In the drawing there are shown some advantageous embodiments of the arrangement according to the invention.

Fig. 1 is a diagram of the metal contents in the lubrication oil in relation to the running operation of the engine. After a driving distance of about 7500 km the concentration of Zn, Pb and Cu in the oil increases suddenly, so that the automotive-engine should be changed and/or repaired.

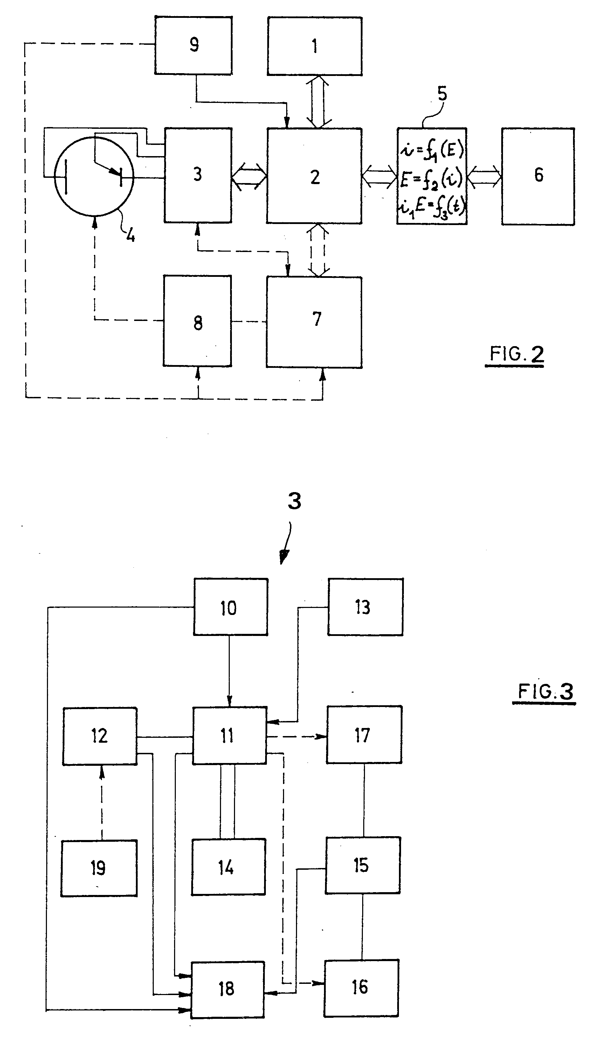

In the arrangement of Fig. 2 a block 1 for setting a program is connected with a block 2 for voltage or current generation. Said block 2 is connected to a block 3 of an electrode detector combined with a measuring system 4 on one hand and a block 5 for sensing, processing and evaluating the signal combined with a block 6 for recording and display on the other hand. According to need, a block 7 for extractor and/or sample treatment is connectable for a determined period with the block 2 for voltage or current generation, with the block 3 of the electrode detector or with a feeding block 9. A block 8 for sample dosing is connected in series or in parallel to said block 7 and to the system 4. The feeding block 9 is connected with the block 2 for voltage or current generation and/or with the block 8 for sample dosing and/or with the block 7 for extractor and/or sample treatment.

As shown in Fig. 3 the block 3 of the electrode detector comprises advantageously a block 10 of setting connected with a block 11 of timing and control. To this block 11 are connected in parallel a block 12 for starting, a block 13 for feeding, a memory block 14 and a block of electrodes 15. The block 11 is further boundable in parallel with a block 16 of electro-mechanical accessory, a block 17 of spare voltage or current outputs and a block 18 of signals and control. Said control block 18 is connected also with the block 10 of setting, the block 12 of starting and/or with the block 11 of timing and control. In certain cases the block 12 for starting is bound independently to a block 19 of external control.

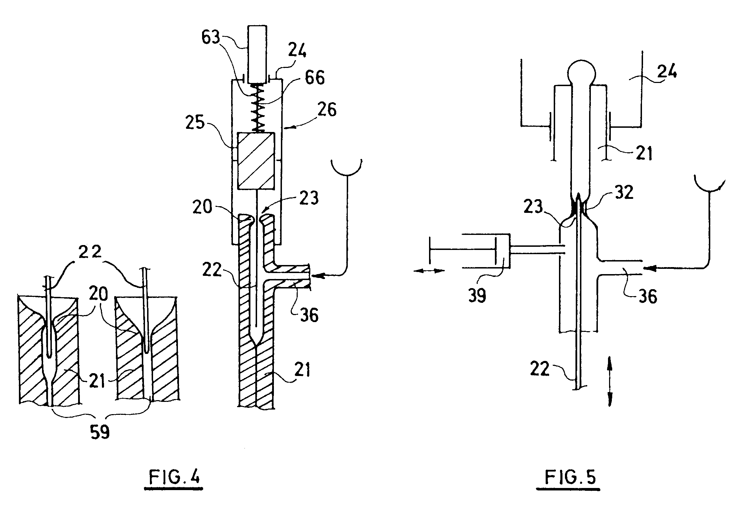

Fig. 4 shows some examples of the block 15 of electrodes containing a measuring electrode 26 where a capillary closure 20 of a capillary member 21 includes a constricting needle 22. The size and shape of said needle 22 correspond to the size and shape of a capillary orifice 23 in the capillary closure 20 or to the size and shape of at least one part of the inner space 59 of the capillary member 21. In the upper part of the body of the measuring electrode 26 there is adjusted a case 24 in which a spring loaded 63, 66 control mechanism 25 supporting the needle 22 is disposed.

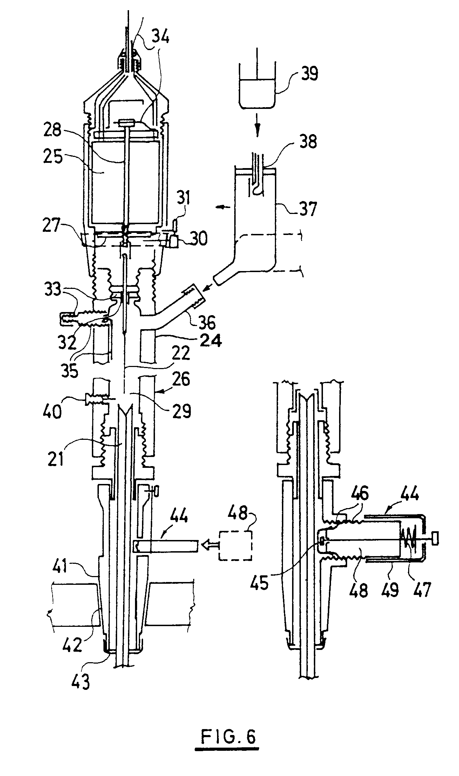

In the arrangements according to Fig. 5 and 6 the block 3 of electrode detector is constructed on the basis of a renewed mercury mini- and microelectrode. As in the example of Fig. 4 in the body of the measuring electrode 26 there is adjusted a case 24 with a fixed control mechanism 25 or with its part and with a sliding mechanism 27. A sliding segment 28 of the control mechanism 25 forms a component of the constricting needle 22 which passes through a closure 32 into the inner reservoir of mercury 29. The wall of the case 24 is provided by a lifting lever mechanism 30 with a lock 31. The needle 22 is connected with the sliding segment 28 of the control mechanism 25 or is adjusted into its shape while at least in the passage through the closure 32 the difference between the outer diameter of the constricting needle 22 and the inner diameter of the closure 32 is less than 0.7 mm and the surface coarseness of the constricting needle 22 in the part passing through the closure 32 does not exceed 0.3 mm. The closure 32 is provided with a packing 33, an electric contact 34 to the constricting needle 22 or to the sliding segment 28, an independent electric contact 35 in the wall of the inner reservoir 29 and with an external mercury reservoir 37 connected to the inlet 36 and provided with a valve 38 or an overpressure doser 39. A stop 40 is formed in the inner reservoir 29. A holding member formed as a reducing piece 41 at the lower part of the body of the measuring electrode 26 is shaped so as to fit its input 42 in a bead or in a wall opening of the arrangement in which the electrode is held. In the lower part of the reducing piece 41 is fixed a packing membrane 43. A mercury drop dislodging device 44 attacks the reducing piece 41, the body of the measuring electrode 26 or the capillary wall 21. For adjusting the amplitude of a hammer 45 an adjustable mechanism 46 is provided which can be shifted along the axis of the hammer 45. The essential parts of the dislodging device 44 are the hammer 45, an elastic element 47, a force element 48 and a case 49 (Fig. 6 right side).

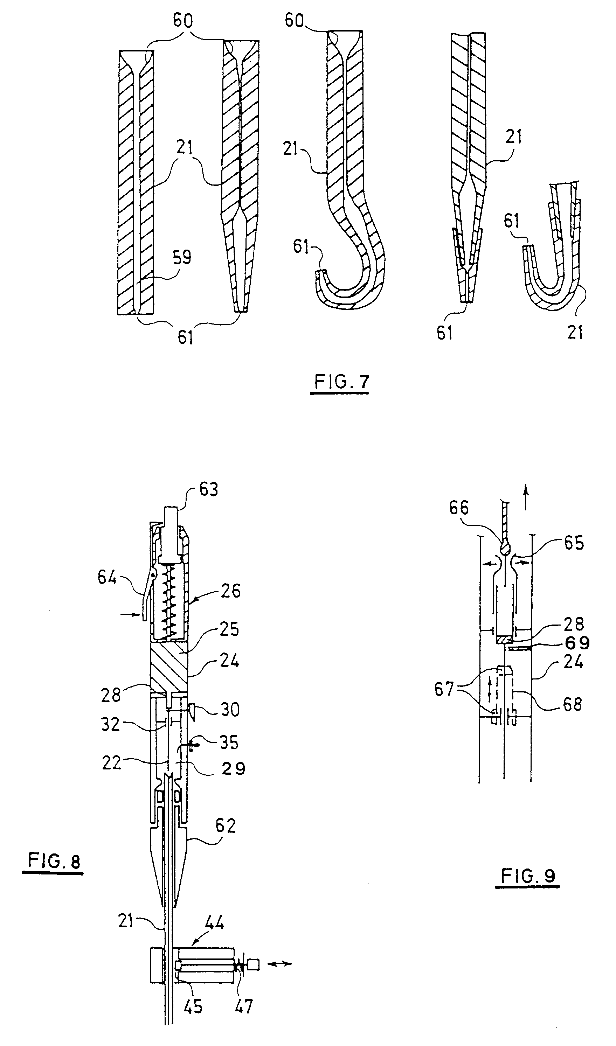

Fig. 7 shows a set of capillaries 21 in which the inner bore 59 in the upper part has the form of a funnel-shaped saddle 60 or of its part, and in the lower part there are, in a given sequence, sections of cylindrical, conically widening and conically narrowing diameters, down to the orifice 61 of the capillaries 21. Other accessories of the arrangement are an evacuation arrangement with an adapter corresponding by its shape and size to the lower end of the capillary 21, the overpressure doser 39, a protective tube for the capillary 21 and a protective case.

The Fig. 8 shows a pen-shaped measuring electrode 26 provided with a clamp 62 of the capillary 21 connected to the inner reservoir 29 with an independent electric contact 35. Above the inner reservoir 29 there is mounted the control mechanism 25 for reproduceable oneway or periodic shifting of the needle 22. Said mechanism 25 is coupled with the mechanism 63 on the basis of a push button, a spring and a limiting stop and a release 64. The parts 24, 28, 30, 44, 45 and 47 have the same function as the corresponding parts in Fig. 6.

As shown in Fig. 9 the control mechanism 25 can include a mechanical system on the basis of the sliding segment 28 with an elastic neck 65. A rod 66 of the starting mechanism is designed in the shape of a pointed cone and disposed above the neck 65. A fixing grip 67 is provided with an elastic segment 68 and an adjustable stop 69.

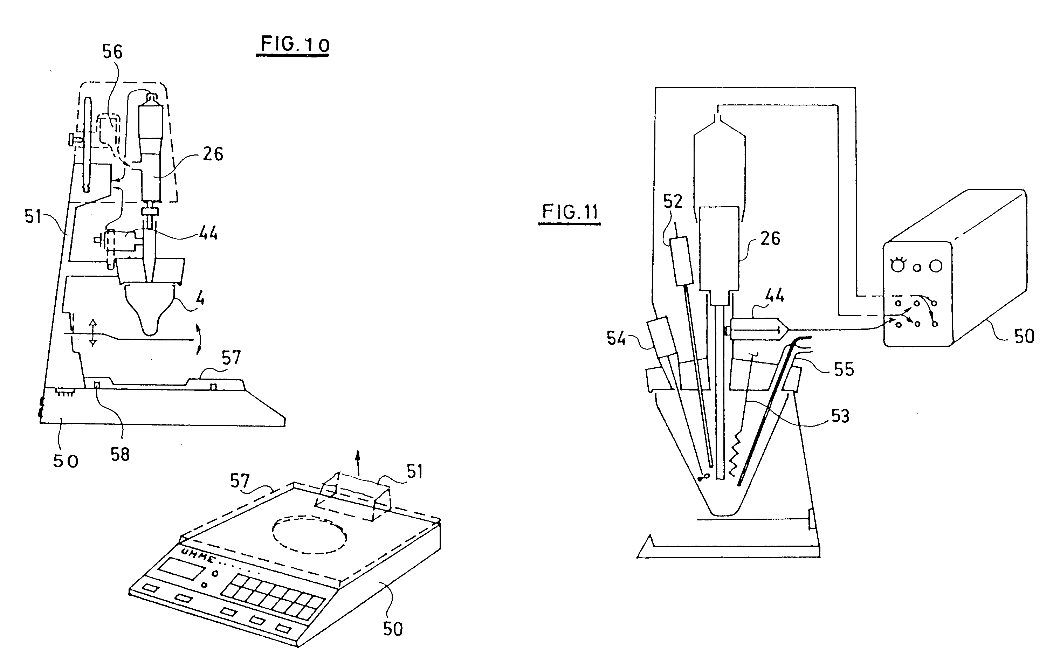

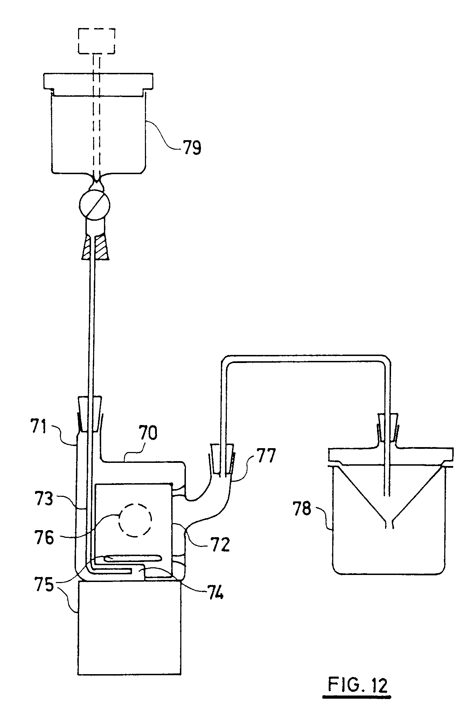

In the apparatus shown in Fig. 10 and 11 the block 10 of setting, the block 11 of timing and control, the block 12 of starting, the memory block 14, the block 18 of signals and control and, according to need, even the block 13 for feeding are designed and disposed in a screened and encased portable controlling block 50 which is connectable to the block 15 of electrodes including the measuring electrode 26, to the drop dislodging device 44 and to a disassemblable stand 51. Clamps are provided for holding the measuring electrode 26, a reference electrode 52, an auxiliary electrode 53, a stirrer 54, a doser of gas 55 chemically inert towards the material of the used system, an external reservoir 56 and, according to need, even the overpressure doser, the measuring system 4, and for fixing a waste pan 57 and a stabilizing support 58.

The apparatus shown in Fig. 12 contains a closed extraction cell 70 provided in its upper part near the wall by an inlet neck 71. A shaped segment 72 is fixed inside the closed extraction cell 70. An inlet tube 73 passes through the inlet neck 71 and ends near a channel 74 close to the bottom of the closed extraction cell 70 below a rotating stirrer 75. In the wall of the shaped segment 72 is formed a slit 76 and an outlet line 77 leads from the side wall of the closed extraction cell 70 to a collecting cell 78. An upper reservoir 79 with a stopcock is connected with the inlet neck 71 and the whole arrangement is supplemented, according to the need, by a mineralizing unit for chemical dissolution of the sample or for its incineration.

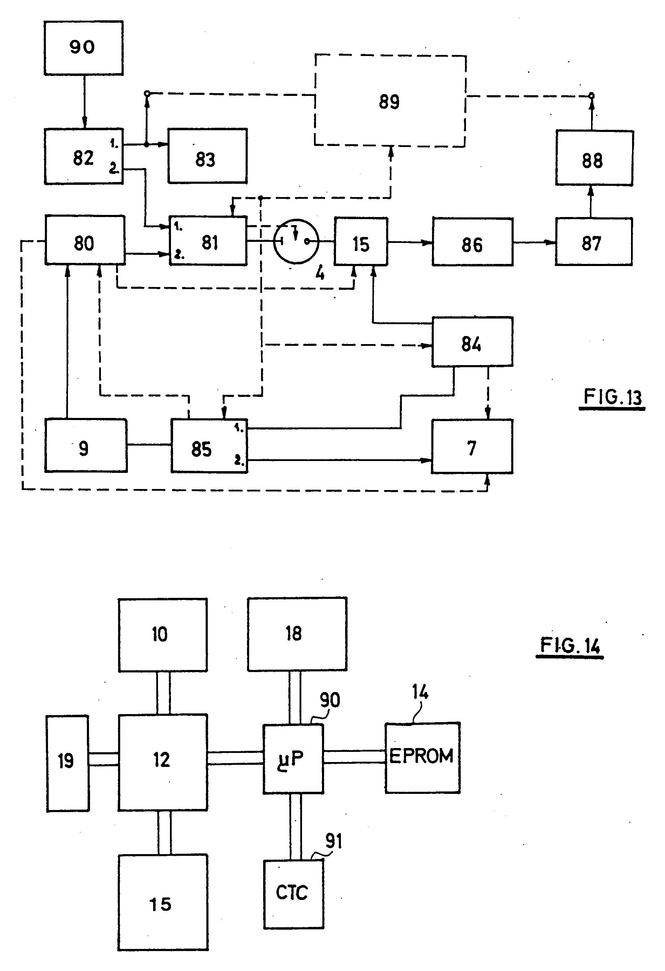

Another example of a more detailed block scheme of the whole system is shown in Fig. 13, where the block for voltage or current generation is formed by a block of timing pulses 80, a block of sum 81, a block of scanning ramp 82 and a block of voltmeter 83. Further the block of the electrode detector contains the block of electrodes 15, a block of programmed electrode control 84 and a block of manual control 85, the block for sensing, processing and evaluating the signal contains a block of interface 86, a block of electronic memories 87 and a block of amplifier and filter output 88. A block 89 of recording, evaluation and control the system includes the block for sensing, processing and evaluating the signal and the block for recording and display. The output of a block of polarity and setting 90 is connected with the block of scanning ramp 82. A first output of said block 82 is led to the input of the block of voltmeter 83 and a second output of said block 82 is connected to first input of the block of sum 81. the outputs of said block of sum 81 are connected to the reference and auxiliary electrodes of the measuring system 4 into which dip in parallel the measuring sensor and the stirrer of the block of electrodes 15. The output of said block 15 is led to the input of the block of interface 86, which is connected to the input of the block of electronic memories 87. The output of said block 87 is led to the input of the block of amplifier and filter output 88. The second input of the block of sum 81 is joined with the output of the block of timing pulses 80. The input of said block 8 is connected with the output of the feeding block 9, which is interconnected with the block of manual control 85. The first output of said block 85 is joined with the input of the block of programmed electrode control 84, the output of which is connected to the input of the block of electrodes 15. the second output of the block of manual control 85 is connected to the block for extractor and/or sample treatment 7.

Fig. 14 shows the example of a block scheme of another electrode detector, in which the block of timing and control contains a block of microprocessor 90 and a block of program generator of time pulses 91. The block of external control 19 is connected with the block for starting 12 which is bound to the block of microprocessor 90 and the memory block 14. Said block for starting 12 is independently connected to the block of setting 10 on the basis of a key-board and to the block of electrodes 15. The block of microprocessor 90 is joined to the block of signals and control 18 and in parallel to the block of program generator of time pulses 91. The block of signals and control 18 consists in that case of an optical part and an acoustic part and the block of external control 19 contains an electronic pulse system started automatically or by a mechanical impulse.

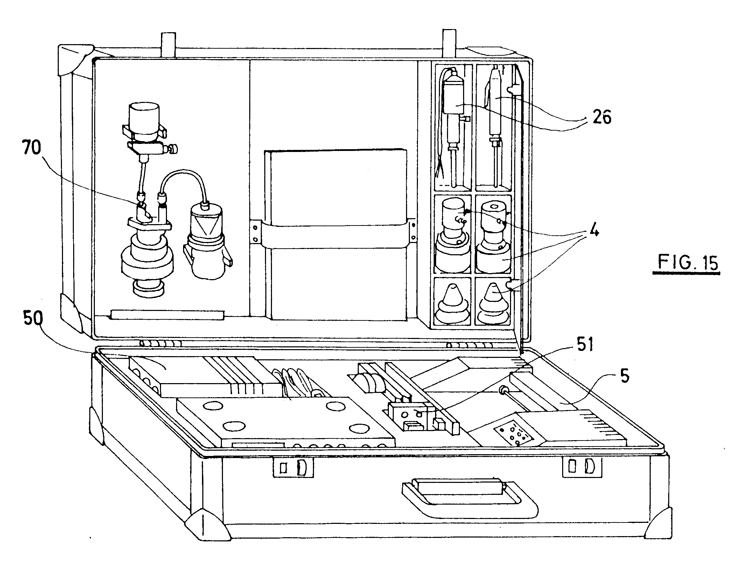

Fig. 15 shows a portable hand-bag for securely packing all elements and parts of the described apparatus.

## Claims

1. Method for the diagnostic of the friction system of a motor on the basis of an analysis of the lubrication oil, in which
- a sample of a lubrication oil used in the motor will be analyzed as to the composition, wear particles ect.,
- the results of said analysis will be compared with the respective known data of an unused lubrication oil, and
- the conditions of the friction system of said motor will be determined on the basis of said comparison,
**characterized** in that
- the sample of used lubrication oil will be mixed with at least one extraction solution having a relative permittivity higher than 4,
- after separating of phases a mercury-based measuring electrode and a reference electrode will be introduced in the resulting extraction solution,
- an electric voltage will be applied in a controlled manner between said measuring electrode and said reference electrode and the electric current passing through the measuring electrode will be measured over a predetermined time interval in dependence on the changes of the applied voltage or
- an electric current will be applied in a controlled manner through said measuring electrode and said reference electrode and the electric potential of the measuring electrode will be measured over a predetermined time interval in dependence of the changes of the applied current, and
- the results of said measurements will be compared to the respectively predetermined data of the unused lubrication oil.

2. Method according to claim 1,
characterized by
- mixing the sample of the lubrication oil repeatedly in predetermined time intervals with the extraction solution,
- measuring the electrical current after each interruption of said mixing the lubrication sample with the extraction solution,
- determining the concentration of
- the individual electrochemical active components and the influence of their oxidation states,
- the compounds having an adsorption coefficient in the extraction solution on the measuring electrode of higher 100 m³/mol and
- the polyaromatic substances on the basis of the measured current values.

3. Method for diagnostic of the friction system of a motor on the basis of an analysis of the lubrication oil, in which
- a sample of a lubrication oil used in the motor will be analyzed as to the composition, wear particles ect.,
- the results of said analysis will be compared with the respective known data of an unused lubrication oil, and
- the conditions of the friction system of said motor will be determined on the basis of said comparison,
**characterized** in that
- a mercury-based micro measuring electrode and a reference electrode will be introduced in a sample of said used lubrication oil,
- an electric voltage will be applied in a controlled manner between said measuring electrode and said reference electrode, the electric current will be measured over a predetermined time interval in dependence of the changes of the applied voltage, or
- an electric current will be applied in a controlled manner through said measuring electrode and the voltage between said measuring electrode and said reference eletrode will be measured over a predetermined time interval in dependence of the changes of the applied current,
- the results of said measurements will be compared to respectively predetermined data of the unused lubrication oil.

4. Method according to claim 1 to 3,
characterized by
- using a mercury-based micro electrode as measuring electrode,
- bringing liquid mercury or mercury amalgam into a capillary measuring member (21),
- shifting a constricting needle (22) alternately in and out of the upper orifice (20) of said capillary member (21), so that the flow of the mercury into said capillary member (21) is reduced, interrupted, renewed or changed in the direction for forming a mercury drop or a mercury meniscus of a required shape and size on the free end of said capillary member (21) in a predetermined time period and
- removing said mercury drop or mercury meniscus after said time period.

5. Apparatus for carrying out the method according to claim 1, 2 or 4,
characterized by
- extractor means (7) comprising a closed extraction cell (70) connected with a extraction solution reservoir (79) by control means and means (75) for generating a stirring action disposed in the extraction cell (70),
- detector means (3) comprising a measuring electrode (26) and a reference electrode (52) connected to an electrical current or voltage source (2),
- a control unit (50) for controlling the electrical current or the voltage supplied to the measuring electrode (26) and
- means (5) for sensing, processing and evaluating the output signals of said detector means (3) combined with means (6) for recording and displaying.

6. Apparatus for carrying out the method according to claim 3,
characterized by
- detector means (3) including a mercury-based micro electrode (26) as measuring electrode and a reference electrode, both dipped in a sample of used lubrication oil and connected with an electrical current or voltage source (2),
- a control unit (50) for controlling the electrical current or the voltage supplied to the measuring micro electrode (26) and
- means (5) for sensing, processing and evaluating the output signals of said measuring means (4) combined with recording and displaying means (6).

7. Apparatus of claim 5 or 6,
characterized in that the measuring electrode (26) comprises a case (24), a capillary member (21) having a capillary closure (20) and a constricting needle (22) connected with a control mechanism (25) disposed in said case (24) for axial shifting the constricting needle (22) into and out of an orifice (23) in said capillary closure (20), the size and shape of said constricting needle (22) corresponds to the size and shape of said capillary orifice (23) or at least of a part of the inner space (59) of the capillary member (21).

8. Apparatus according to claim 6 or 7,
characterized in that
the inner bore (59) of the capillary member (21) is formed at its upper end portion as a funnel-shaped saddle (60) and is provided with a widened section in a lower portion and with an outlet opening (61) on its lower end or in an adapter.

9. Apparatus according to claim 7,
characterized in that
- an inner mercury reservoir (29) is disposed in the case (24) and
- the constricting needle (22) is connected to a sliding element (28) of the control mechanism (25), which passes through a closure (32) into said mercury reservoir (29), the difference between the needle diameter and the inner diameter of said closure (32) is less than 0.7 mm and the surface coarseness of the needle (22) in the part passing through said closure (32) is less than 0.3 mm.

10. Apparatus according to claim 9,
characterized in that
the closure (32) is provided with a packing means (33), with an electric contact (34) to the sliding member (28) and with an independent electric contact (35) on the wall of the mercury reservoir (29), and said reservoir (29) is provided with an inlet (36) for connecting with an outer mercury reservoir (37).

11. Apparatus according to claim 9 or 10,
characterized in that
a stopping means (40) is disposed in the mercury reservoir (29) and a holding member (41) is mounted on the lower portion of the
measuring electrode (26).

12. Apparatus according to claim 11,
characterized in that
in the measuring electrode (26) it is provided a mercury drop dislodging device (44) comprising a hammer (45), elastic element (47) and forcing elements (48) for moving said hammer (45).

13. Apparatus according to claim 5 to 12,
characterized in that
the pan-shaped measuring electrode (26) comprises a clamp (62) for holding the capillary member (21) connected to the inner reservoir (29) and that the control mechanism (25) mounted above the inner reservoir (29) includes a mechanism (63) for shifting the constricting needle (22) and a releasing member (64).

14. Apparatus according to claim 8 to 13,
characterized in that
the sliding element (28) of the control mechanism (25) is provided with an elastic neck (65) for detachable gripping a rod (66) of the pushing member (63) and with a fixing grip (67) combined with a spring element (68).

15. Apparatus according to claim 5 to 14,
characterized in that
the control unit (50) is designed as a portable controlling block and includes setting means (10), timing and control means (11), starting means (12), memory means (14) and signal means (18).

16. Apparatus according to claim 15,
characterized in that
the supporting frame (52) is detachable mounted on a box-shaped housing of the control unit (50).

17. Apparatus according to at least one of the claims 5, 7 to 16,
characterized in that
- the closed extraction cell (70) of the extractor mean (7) is provided in its upper portion near the wall by an inlet neck (71) for holding an inlet tube (73) which ends in a channel (74) near to the cell bottom and to a rotating stirrer (74),
- in the wall of a shaped element (72) fixed in the extraction cell (70) is formed a slit (76),
- an outlet (77) in the sidewall of the extraction cell (70) leads to a collecting cell (78),
- an upper reservoir (79) with a stopcock as control means is connected with the inlet tube (73) and
- the whole arrangement can be supplemented by a mineralizing unit for chemical dissolution of the sample or for its incineration.

18. Apparatus according to at least one of the claims 5 to 17,
characterized in that
all elements and parts are securely packed in a specially designed portable container.

## Patentansprüche

1. Verfahren zur Diagnose des Reibungssystems von Motoren auf der Basis einer Analyse des Schmieröls, worin
- eine Probe eines im Motor verwendeten Schmieröls in Bezug auf die Zusammensetzung, Verschleißpartikel usw. analysiert wird,
- die Ergebnisse der Analyse mit den entsprechenden bekannten Daten eines nicht gebrauchten Schmieröls verglichen werden und
- der Zustand des Reibungssystems des Motors auf der Basis dieses Vergleichs bestimmt wird
**dadurch gekennzeichnet,** daß
- die Probe des gebrauchten Schmieröls mit mindestens einer Extraktionslösung vermischt wird, die eine relative Dielektrizitätskonstante über 4 aufweist,
- nach der Phasentrennung eine auf der Basis von Quecksilber gebildete Meßelektrode und eine Referenzelektrode in die sich ergebende Extraktionslösung eingeführt werden,
- eine gesteuerte elektrische Spannung zwischen der Meßelektrode und der Referenzelektrode angelegt wird, und der elektrische Strom, der durch die Meßelektrode hindurchgeht, einen vorgegebenen Zeitraum lang in Abhängigkeit von den Schwankungen der angelegten Spannung gemessen wird oder
- durch die Meßelektrode und die Referenzelektrode ein gesteuerter elektrischer Strom geleitet und das elektrische Potential der Meßelektrode einen vorgegebenen Zeitraum lang in Abhängigkeit von den Schwankungen des angelegten Stroms gemessen wird, und
- die Ergebnisse der Messungen mit den entsprechenden vorgegebenen Daten des nicht gebrauchten Schmieröls verglichen werden.

2. Verfahren gemäß Anspruch 1, gekennzeichnet durch
- wiederholtes Mischen der Probe des Schmieröls mit der Extraktionslösung in vorgegebenen Zeitabständen,
- Messen des elektrischen Stroms nach jeder Unterbrechung des Mischens der Schmierprobe mit der Extraktionslösung,
- Bestimmen der Konzentration
- der individuellen elektrochemisch wirksamen Komponenten und des Einflusses ihrer Oxidationszustände,
- der Verbindungen, die einen Adsorptionskoeffizienten in der Extraktionslösung an der Meßelektrode über 100 m³/mol aufweisen, und
- der polyaromatischen Substanzen auf der Basis der gemessenen Stromwerte.

3. Verfahren zur Diagnose des Reibungssystems von Motoren auf der Basis einer Analyse des Schmieröls, worin
- eine Probe eines im Motor verwendeten Schmieröls in Bezug auf die Zusammensetzung, Verschleißpartikel usw. analysiert wird;
- die Ergebnisse der Analyse mit den entsprechenden bekannten Daten eines nicht gebrauchten Schmieröls verglichen werden, und
- der Zustand des Reibungssystems des Motors auf der Basis dieses Vergleichs bestimmt wird
**dadurch gekennzeichnet,** daß
- eine auf der Basis von Quecksilber gebildete Micromeßelektrode und eine Referenzelektrode in eine Probe gebrauchten Schmieröls eingeführt werden,
- eine gesteuerte elektrische Spannung zwischen der Meßelektrode und der Referenzelektrode angelegt, und der elektrische Strom einen vorgegebenen Zeitraum lang in Abhängigkeit von den Schwankungen der angelegten Spannung gemessen wird, oder
- durch die Meßelektrode ein gesteuerter elektrischer Strom geleitet und die Spannung zwischen Meßelektrode und Referenzelektrode einen vorgegebenen Zeitraum lang in Abhängigkeit von den Schwankungen des angelegten Stroms gemessen wird, und
- die Ergebnisse der Messungen mit den entsprechenden vorgegebenen Daten von nicht gebrauchtem Schmieröl verglichen werden.

4. Verfahren gemäß Ansprüchen 1 bis 3, gekennzeichnet durch
- Verwenden einer auf der Basis von Quecksilber gebildeten Micromeßelektrode als Meßelektrode,
- Einbringen von flüssigem Quecksilber oder Quecksilberamalgam in ein Kapillar-Meßelement (21),
- Verschieben einer sich verjüngenden Nadel (22) in der oberen Öffnung (20) des Kapillarelements (21) wechselweise hinein und heraus, so daß der Zufluß des Quecksilbers in das Kapillarelement (21) zum Bilden eines Quecksilbertropfens oder eines Quecksilbermeniskus einer erwünschten Form und Größe auf dem freien Ende des Kapillarelements (21) in einem vorgegebenen Zeitraum verringert, unterbrochen, erneuert oder geändert wird, und
- Entfernen des Quecksilbertropfens oder des Quecksilbermeniskus nach Ablauf des genannten Zeitraums.

5. Vorrichtung zum Durchführen des Verfahrens gemäß Anspruch 1, 2 oder 4, gekennzeichnet durch
- eine Extraktionseinrichtung (7) mit einer geschlossenen Extraktionszelle (70), die über eine Steuereinheit und eine Einrichtung (75) zum Erzeugen eines Rührbetriebs, die in der Extraktionszelle (70) angeordnet ist, mit einem Extraktionslösungs-Reservoir (79) in Verbindung steht,
- eine Detektoreinheit (3), die eine Meßelektrode (26) und eine Referenzelektrode (52) umfaßt, die mit einer Stromquelle oder einer spannungsquelle (2) verbunden sind,
- eine Steuereinheit (50) zum Steuern des elektrischen Stroms oder der Spannung, der/die der Meßelektrode (26) zugeführt wird, und
- eine Einheit (5) zum Erfassen, Verarbeiten und Beurteilen der von der Detektoreinheit (3) ausgegebenen Signale, die mit einer Einheit (6) zum Aufzeichnen und Anzeigen kombiniert ist.

6. Vorrichtung zur Durchführung des Verfahrens gemäß Anspruch 3, gekennzeichnet durch
- eine Detektoreinheit (3) einschließlich einer Quecksilber-Microelektrode (26) als Meßelektrode und einer Referenzelektrode, die beide in eine Probe gebrauchten Schmieröls eingetaucht werden und mit einer elektrischen Stromquelle oder einer Spannungsquelle (2) verbunden werden,
- eine Steuereinheit (50) zum Steuern des der Micromeßelektrode (26) zugeführten Stroms oder der Spannung und
- eine Einheit (5) zum Erfassen, Verarbeiten und Beurteilen der von der Meßeinheit (4) ausgegebenen Signale, die mit einer Einheit (6) zum Aufzeichnen und Anzeigen kombiniert ist.

7. Vorrichtung gemäß Anspruch 5 oder 6, dadurch gekennzeichnet, daß die Meßelektrode (26) ein Gehäuse (24), ein Kapillarelement (21) mit einem Kapillarverschluß (20) und einer sich verjüngenden Nadel (22) umfaßt, und das mit einem Steuermechanismus (25) verbunden ist, der zum axialen Verschieben der sich verjüngenden Nadel (22) in eine Öffnung (23) in den Kapillarverschluß (20) hinein und aus dieser heraus im Gehäuse (24) angeordnet ist, wobei die Größe und Form der sich verjüngenden Nadel (22) der Größe und Form der Kapillaröffnung (23) oder zumindest eines Teiles des Innenraums (59) des Kapillarelements (21) entspricht.

8. Vorrichtung gemäß Anspruch 6 oder 7, dadurch gekennzeichnet, daß die Innenbohrung (59) des Kapillarelements (21) in ihrem oberen Endabschnitt als trichterförmiger Sattel (60) ausgebildet ist, in ihrem unteren Abschnitt ein erweitertes Teilstück und im unteren Endabschnitt oder in einem Adapter eine Auslaßöffnung (61) aufweist.

9. Vorrichtung gemäß Anspruch 7, dadurch gekennzeichnet, daß
- ein inneres Quecksilberreservoir (29) im Gehäuse (24) angeordnet ist, und
- die sich verjüngende Nadel (22) mit einem Gleitelement (28) des Steuermechanismus (25) verbunden ist, das durch einen Verschluß (32) hindurch- und in das Quecksilberreservoir (29) hineingeht, wobei der Unterschied zwischen dem Nadeldurchmesser und dem Innendurchmesser des Verschlusses (32) weniger als 0,7 mm beträgt und die Oberflächenrauhigkeit der Nadel (22) in dem Abschnitt, der durch den Verschluß (32) hindurchgeht, unter 0,3 mm liegt.

10. Vorrichtung gemäß Anspruch 9, dadurch gekennzeichnet, daß der Verschluß (32) mit einer Abdichtung (33), einem elektrischen Kontakt (34) zum Gleitelement (28) und mit einem unabhängigen elektrischen Kontakt (35) auf der Wand des Quecksilberreservoirs (29) ausgestattet ist, und daß das Reservoir (29) mit einem Einlaß (36) zur Verbindung mit einem externen Quecksilberreservoir (37) ausgestattet ist.

11. Vorrichtung gemäß Anspruch 9 oder 10, dadurch gekennzeichnet, daß ein Anschlag (40) im Quecksilberreservoir (29) ausgebildet ist, und daß ein Halteelement (41) am unteren Abschnitt der Meßelektrode (26) montiert ist.

12. Vorrichtung gemäß Anspruch 11, dadurch gekennzeichnet, daß in der Meßelektrode (26) eine Ausgabevorrichtung (44) für einen Quecksilbertropfen mit einem Hammer (45), einem elastischen Element (47) und Druckelementen (48) zum Bewegen des Hammers (45) vorgesehen ist.

13. Vorrichtung gemäß den Ansprüchen 5 bis 12, dadurch gekennzeichnet, daß die Meßelektrode (26) in Pfannenform eine Klemme (62) zum Halten des Kapillarelements (21) umfaßt, die mit dem inneren Reservoir (29) verbunden ist, und daß der Steuermechanismus (25), der über dem inneren Reservoir (29) angeordnet ist, einen Mechanismus (63) zum Verschieben der sich verjüngenden Nadel (22) und ein Auslöseelement (64) umfaßt.

14. Vorrichtung gemäß den Ansprüchen 8 bis 13, dadurch gekennzeichnet, daß das Gleitelement (28) des Steuermechanismus (25) zum abnehmbaren Ergreifen einer Stange (66) des Schiebemechanismus (63) mit einem elastischen Hals (65) und, kombiniert mit einem Federelement (68), mit einem feststellbaren Greifer (67) ausgestattet ist.

15. Vorrichtung gemäß den Ansprüchen 5 bis 14, dadurch gekennzeichnet, daß die Steuereinheit (50) tragbar ist und eine Einstelleinrichtung (10), eine Einheit zum Timing und zur Steuerung (11), eine Starteinheit (12), eine Speichereinheit (14) und eine Signaleinheit (18) umfaßt.

16. Vorrichtung gemäß Anspruch 15, dadurch gekennzeichnet, daß der Stützrahmen (52) abnehmbar auf einem kastenförmigen Gehäuse der Steuereinheit (50) ausgebildet ist.

17. Vorrichtung gemäß mindestens einem der Ansprüche 5, 7 bis 16, dadurch gekennzeichnet, daß
- im oberen Abschnitt der geschlossenen Extraktionszelle (70) der Extraktionseinrichtung (7) in der Nähe der Wand ein Hals (71) zum Halten eines Einlaßrohrs (73) ausgebildet ist, das in einem Kanal (74) in der Nähe des Zellenbodens und eines drehbaren Rührers (74) endet,
- in der Wand eines in der Extraktionszelle befestigten geformten Elements (72) ein Schlitz (76) ausgeführt ist,
- ein Auslaß (77) in der Seitenwand der Extraktionszelle (70) zu einer Sammelzelle (78) führt,
- ein oberes Reservoir (79) mit einem Absperrhahn als Steuereinrichtung mit dem Einlaßrohr (73) verbunden ist, und
- die gesamte Anordnung durch eine Einheit für ein anorganisches Aufschlußmittel zum Aufschluß der Probe unter chemischer Auflösung oder zum Veraschen der Probe ergänzt werden kann.

18. Vorrichtung gemäß mindestens einem der Ansprüche 5 bis 17, dadurch gekennzeichnet, daß alle Elemente und Teile sicher in einem speziell dafür konstruierten tragbaren Behälter verpackt sind.

## Revendications

1. Procédé de diagnostic d'un système à friction de moteur sur la base d'une analyse de l'huile de lubrification, dans lequel :
- un échantillon d'huile de lubrification usagée du moteur est analysé en ce qui concerne la composition, les particules d'usure, etc.,
- les résultats de ladite analyse sont comparés aux données connues respectives d'une l'huile de lubrification non-usagée, et
- les états du système à friction dudit moteur sont déterminés sur la base de ladite comparaison,
caractérisé en ce que :
- l'échantillon d'huile de lubrification usagée est mélangé avec au moins une solution d'extraction ayant une permissivité relative plus élevée que 4,
- après séparation des phases une électrode de mesure à base de mercure et une électrode de référence sont introduites dans la solution d'extraction résultante,
- une tension électrique est appliquée de manière commandée entre ladite électrode de mesure et ladite électrode de référence et le courant électrique passant à travers l'électrode de mesure est mesuré sur un intervalle de temps prédéterminé en fonction des changements de tension appliquée ou,
- un courant électrique est appliqué de manière commandée à travers ladite électrode de mesure et ladite électrode de référence et le potentiel électrique de l'électrode de mesure est mesuré sur un intervalle de temps prédéterminé en fonction des changements de courant appliqué, et
- les résultats desdites mesures sont comparés aux données respectivement prédéterminées de l'huile de lubrification non-usagée.

2. Procédé selon la revendication 1, caractérisé en ce qu'il comporte les étapes consistant à :
- mélanger l'échantillon d'huile de lubrification de manière répétée dans des intervalles de temps prédéterminés avec la solution d'extraction,
- mesurer le courant électrique après chaque interruption dudit mélange de l'échantillon de lubrification avec la solution d'extraction,
- déterminer la concentration :
- des composants électrochimiques actifs individuels et l'influence de leurs états d'oxydation,
- des compositions ayant un coefficient d'adsorption de la solution d'extraction sur l'électrode de mesure qui est plus élevé que 100 m³/mol, et
- des substances polyaromatiques sur la base des valeurs de courant mesurées.

3. Procédé de diagnostic d'un système à friction de moteur sur la base d'une analyse de l'huile de lubrification, dans lequel :
- un échantillon d'huile de lubrification usagée du moteur est analysé en ce qui concerne la composition, les particules d'usure, etc.,
- les résultats de ladite analyse sont comparés aux données connues respectives d'une huile de lubrification non-usagée, et
- les états du système à friction dudit moteur sont déterminés sur la base de ladite comparaison,
caractérisé en ce que :
- une électrode de micro-mesure à base de mercure et une électrode de référence sont introduites dans un échantillon de ladite huile de lubrification usagée,
- une tension électrique est appliquée de manière commandée entre ladite électrode de mesure et ladite électrode de référence, le courant électrique est mesuré sur un intervalle de temps prédéterminé en fonction des changements de tension appliquée ou,
- un courant électrique est appliqué de manière commandée à travers ladite électrode de mesure et ladite électrode de référence et la tension entre ladite électrode de mesure et ladite électrode de référence est mesurée sur un intervalle de temps prédéterminé en fonction des changements de courant appliqué, et
- les résultats desdites mesures sont comparés aux données respectivement prédéterminées de l'huile de lubrification non-usagée.

4. Procédé selon l'une quelconque des revendications 1 à 3, caractérisé en ce qu'il comporte les étapes consistant à :
- utiliser une micro-électrode à base de mercure en tant qu'électrode de mesure,
- apporter du mercure liquide ou un amalgame de mercure à l'intérieur d'un élément de mesure capillaire (21),
- déplacer une aiguille d'étranglement (22) de manière alternée dans l'orifice supérieur (20) et à l'extérieur de l'orifice supérieur dudit élément capillaire (21), de sorte que l'écoulement du mercure à l'intérieur dudit élément capillaire (21) soit réduit, interrompu, renouvelé ou changé dans la direction destinée à former une goutte de mercure ou un ménisque de mercure ayant une forme et une dimension requises sur l'extrémité libre dudit élément capillaire (21) dans une période de temps prédéterminée, et
- enlever ladite goutte de mercure ou ledit ménisque de mercure après ladite période de temps.

5. Dispositif pour mettre en oeuvre le procédé selon la revendication 1, 2 ou 4, caractérisé en ce qu'il comporte :
- des moyens (7) d'extraction comportant une cellule d'extraction fermée (70) reliée à un réservoir (79) de solution d'extraction par des moyens de commande et par des moyens (75) destinés à engendrer une action d'agitation agencés dans la cellule d'extraction (70),
- des moyens de détection (3) comportant une électrode de mesure (26) et une électrode de référence (52) reliées à une source (2) de courant ou de tension électrique,
- un ensemble de commande (50) destiné à commander le courant, ou la tension, électrique alimenté vers l'électrode de mesure (26) et
- des moyens (5) pour détecter, traiter et évaluer les signaux de sortie desdits moyens de détection (2) combinés à des moyens (6) d'enregistrement et d'affichage.

6. Dispositif pour mettre en oeuvre le procédé selon la revendication 3, caractérisé en ce qu'il comporte :
- des moyens de détection (3) comportant une micro-électrode (26) à base de mercure en tant qu'électrode de mesure et une électrode de référence, toutes deux étant plongées dans un échantillon d'huile de lubrification usagée et reliées à une source (2) de courant ou de tension électrique,
- un ensemble de commande (50) destiné à commander le courant, ou la tension, électrique envoyé à la micro-électrode de mesure (26), et
- des moyens (5) pour détecter, traiter et évaluer les signaux de sortie desdits moyens de mesure (4) combinés à des moyens d'enregistrement et d'affichage (6).

7. Dispositif selon la revendication 5 ou 6, caractérisé en ce que l'électrode de mesure (26) comporte un boîtier (24), un élément capillaire (21) ayant une enceinte capillaire (20) et une aiguille d'étranglement (22) reliée à un mécanisme de commande (25) agencé dans ledit boîtier (24) pour déplacer axialement l'aiguille d'étranglement (22) vers l'intérieur et vers l'extérieur d'un orifice (23) de ladite enceinte (20) de capillaire, la dimension et la forme de ladite aiguille d'étranglement (22) correspondent à la dimension et la forme dudit orifice capillaire (23) ou au moins d'une partie de l'espace intérieur (59) de l'élément capillaire (21).

8. Dispositif selon la revendication 6 ou 7, caractérisé en ce que l'alésage intérieur (59) de l'élément capillaire (21) est formé au niveau de sa partie d'extrémité supérieure sous forme d'une selle (60) en forme d'entonnoir et est muni d'un tronçon élargi dans une partie inférieure et d'une ouverture de sortie (61) sur son extrémité inférieure ou dans un adaptateur.

9. Dispositif selon la revendication 7, caractérisé en ce que :
- un réservoir intérieur de mercure (29) est agencé dans le boîtier (24), et
- l'aiguille d'étranglement (22) est reliée à un élément coulissant (28) du mécanisme de commande (25), qui passe à travers une enceinte (32) jusqu'à l'intérieur dudit réservoir de mercure (29), la différence entre le diamètre de l'aiguille et le diamètre intérieur de ladite enceinte (32) est plus petite que 0,7 mm et la rugosité superficielle de l'aiguille (22) dans la partie passant à travers ladite enceinte (32) est plus petite que 0,3 mm.

10. Dispositif selon la revendication 9, caractérisé en ce que l'enceinte (32) est munie de moyens (33) formant garniture, d'un contact électrique (34) pour l'élément coulissant (28) et d'un contact électrique indépendant (35) situé sur la paroi du réservoir de mercure (29), et ledit réservoir (29) est muni d'une entrée (36) destinée à être reliée à un réservoir extérieur de mercure (37).

11. Dispositif selon la revendication 9 ou 10, caractérisé en ce que des moyens d'arrêt (40) sont agencés dans le réservoir de mercure (29) et un élément de support (41) est monté sur la partie inférieure de l'électrode de mesure (26).

12. Dispositif selon la revendication 11, caractérisé en ce que dans l'électrode de mesure (26) est agencé un dispositif (44) de séparation de goutte de mercure comportant un marteau (45), un élément élastique (47) et des éléments de transmission de force (48) destinés à déplacer ledit marteau (45).

13. Dispositif selon l'une quelconque des revendications 5 à 12, caractérisé en ce que l'électrode de mesure (26) en forme de bac comporte une attache (62) destinée à maintenir le tube capillaire (21) relié au réservoir intérieur (29) et en ce que le mécanisme de commande (25) monté au-dessus du réservoir intérieur (29) comporte un mécanisme (63) pour déplacer l'aiguille d'étranglement (22) et un élément de libération (64).

14. Dispositif selon l'une quelconque des revendications 8 à 13, caractérisé en ce que l'élément coulissant (28) du mécanisme de commande (25) est muni d'un col élastique (65) pour saisir de manière séparable une tige (66) de l'élément de poussée (63), et d'un élément de fixation (67) combiné à un élément (68) formant ressort.

15. Dispositif selon l'une quelconque des revendications 5 à 14, caractérisé en ce que l'ensemble de commande (50) est conçu en tant que bloc de commande portable et comporte des moyens de mise en oeuvre (70), des moyens de synchronisation et de commande (11), des moyens de démarrage (12), des moyens (14) formant mémoire et des moyens de signaux (18).

16. Dispositif selon la revendication 15, caractérisé en ce que le cadre de support (52) est monté de manière à pouvoir être séparé sur un boîtier en forme de boîte de l'ensemble de commande (50).

17. Dispositif selon l'une quelconque des revendications 5, 7 à 16, caractérisé en ce que :
- la cellule d'extraction fermée (70) des moyens d'extraction (7) est munie dans sa partie supérieure, à proximité de la paroi, d'un col d'entrée (71) destiné à retenir un tube d'entrée (73) qui se termine dans un canal (74) situé à proximité du fond de la cellule et d'un agitateur rotatif (75),
- dans la paroi d'un élément (72) mis en forme fixé dans la cellule d'extraction (70) est formée une fente (76),
- une sortie (77) agencée dans la paroi latérale de la cellule d'extraction (70) aboutit à une cellule collectrice (78),
- un réservoir supérieur (79) ayant une soupape d'arrêt en tant que moyens de commande est relié au tube d'entrée (73) et
- l'agencement dans son entier peut être muni de plus d'un ensemble minéralisateur pour dilution chimique de l'échantillon ou pour incinération de celui-ci.

18. Dispositif selon l'une quelconque des revendications 5 à 17, caractérisé en ce que tous les éléments et toutes les parties sont emballées de manière sûre dans un conteneur portable conçu de manière spécifique.
